# FASCICULE DE BREVET EUROPEEN

(11) **EP 3 814 311 B1**
(45) Date de publication et mention de la délivrance du brevet: **23.04.2025**
(21) Numéro de dépôt: 19746124.7
(22) Date de dépôt: 21.06.2019
(51) Int. Cl.: C07C 17/20, C07C 21/18

(54) **PROCÉDÉ DE PRODUCTION DU 1-CHLORO-3,3,3-TRIFLUOROPROPÈNE**
VERFAHREN ZUR HERSTELLUNG VON 1-CHLOR-3,3,3-TRIFLUORPROPEN
METHOD FOR PRODUCING 1-CHLORO-3,3,3-TRIFLUOROPROPENE

(30) Priorité: 27.06.2018 FR 1855793
(43) Date de publication de la demande: 05.05.2021
(73) Titulaire: ARKEMA FRANCE, 92700 Colombes (FR)
(72) Inventeur: HISLER, Kevin, 69491 Pierre-Benite Cedex (FR); PIGAMO, Anne, 69491 Pierre-Benite Cedex (FR); WENDLINGER, Laurent, 69491 Pierre-Benite Cedex (FR); BOUSSARIE, Emmanuel, 69491 Pierre-Benite Cedex (FR)
(74) Mandataire: Arkema Patent
(86) Numéro de dépôt international: PCT/FR2019/051520
(87) Numéro de publication internationale: WO 2020/002800

(56) Documents cités:
- WO-A1-2015/104517
- US-A- 5 684 219
- US-A1- 2013 261 353
- US-A1- 2013 261 354
- US-A1- 2014 221 704

## Description

### Domaine technique de l'invention

La présente invention concerne la production d'hydrochlorofluorooléfines. Plus particulièrement, la présente invention concerne la production de 1-chloro-3,3,3-trifluoroproprène.

### Arrière-plan technologique de l'invention

Le 3,3,3-trifluoro-1-chloropropène ou encore 1-chloro-3,3,3-trifluoropropène (HCFO-1233zd) existe sous forme de deux isomères : l'isomère cis, à savoir le Z-3,3,3-trifluoro-1-chloropropène (HCFO-1233zdZ), et l'isomère trans, à savoir le E-3,3,3-trifluoro-1-chloropropène (HCFO-1233zdE). Ils ont des points d'ébullition différents, respectivement de 18,5°C pour le composé trans et de 39,5°C pour le composé cis.

Les fluides à base de E-3,3,3-trifluoro-1-chloropropène (HCFO-1233zdE) ont trouvé de nombreuses applications dans des domaines industriels variés, notamment en tant que fluides de transfert de chaleur, propulseurs, agents moussants, agents gonflants, diélectriques gazeux, milieux de polymérisation ou monomères, fluides supports, agents abrasifs, agents de séchage et fluides pour unité de production d'énergie.

La fabrication du HCFO-1233zdE s'accompagne d'une multitude de sous-produits, ayant un point d'ébullition proche du HCFO-1233zdE. Cela conduit à des étapes de purification assez complexes et coûteuses. Les difficultés rencontrées au cours de la purification du HCFO-1233zdE impliquent généralement une perte conséquente en produit recherché. De plus, les sous-produits peuvent former des compositions azéotropiques avec le HCFO-1233zdE, rendant très difficile voire impossible la séparation par distillation simple.

On connait par US 5,877,359 un procédé de préparation du HCFO-1233zdE à partir du 1,1,3,3-tétrachloropropène en phase liquide et en l'absence de catalyseur. Le ratio molaire HF/1230za dans le réacteur de fluoration est de 12 à 500. On connait également par US 9,643,903 un procédé de fluoration du 1,1,3,3-tétrachloropropène en phase liquide et en l'absence de catalyseur dans un milieu riche en HF. On connait également par US2013/261354 un procédé de préparation du 1-chloro-3,3,3-trifluoropropène à partir d'un mélange 1,1,3,3-tétrachloropropène et 1,3,3,3-tétrachloropropène. On connait également par WO2015/104517 un procédé de préparation du E-1-chloro-3,3,3-trifluoropropène à partir du 1,1,3,3-tétrachloropropène.

Par ailleurs, on observe une quantité de sous-produits surfluorés non négligeables liés à la présence de cet HF en grande quantité. La présence de 245fa peut entrainer une perte de rendement car il est connu que ce mélange forme un mélange azéotropique avec le produit principal, le 1233zdE (voir notamment US2017/174965). Il sera donc difficile à séparer et devra être éliminé sous forme de mélange azéotropique, entrainant ainsi une perte de rendement.

Il existe donc un besoin pour de nouveaux procédés minimisant les inconvénients décrits ci-dessus.

### Résumé de l'invention

La présente invention concerne un procédé de production de 1-chloro-3,3,3-trifluoropropène (HCFO-1233zd) comprenant les étapes de :
i) la fourniture :
   - d'un courant **A1** comprenant HF,
   - d'un courant **A2** comprenant 1,1,3,3-tetrachloropropene et/ou 1,3,3,3-tetrachloropropene, et
   - d'un réacteur contenant une phase liquide,
ii) dans ledit réacteur, la mise en contact dans ladite phase liquide et en l'absence d'un catalyseur dudit courant **A1** comprenant HF avec ledit courant **A2** comprenant 1,1,3,3-tetrachloropropene et/ou 1,3,3,3-tetrachloropropene dans des conditions suffisantes pour produire un courant C comprenant 1-chloro-3,3,3-trifluoropropène, HF et HCl,
iii) la récupération dudit courant **C,**
caractérisé en ce que ledit réacteur est pourvu de moyens d'agitation de ladite phase liquide de sorte à maintenir pendant la mise en œuvre de l'étape ii), la température de ladite phase liquide varie d'au maximum 2°C. Le(s) moyen(s) d'agitation permet(tent) un bon contact entre les deux matières premières immiscibles, i.e. HF et le 1,1,3,3-tetrachloropropene et/ou 1,3,3,3-tetrachloropropene. La présence de moyens d'agitation permet d'éviter les phénomènes de décantation qui pourraient perturber le processus réactionnel. Il a été découvert que lorsque la température de la phase liquide est constante ou substantiellement constante entre la surface du volume liquide et le fond du réacteur au cours du processus réactionnel, ladite phase liquide et les deux matières premières présentent une très bonne homogénéité permettant de limiter la formation de coproduits.

Le présent procédé permet, grâce au contrôle de la température de la phase liquide, la production de 1-chloro-3,3,3-trifluoropropène tout en minimisant la formation de coproduits surfluorés ou de composés lourds (dimères ou trimères de composés en C3).

Selon un mode de réalisation préféré, lesdits moyens d'agitation comprennent un mélangeur statique, un dispositif d'injection d'un gaz inerte dans ladite phase liquide, un dispositif de recirculation de la phase liquide ou un dispositif de reflux de HF dans ladite phase liquide ou une combinaison de plusieurs de ces moyens d'agitation.

Selon un mode de réalisation préféré, le moyen d'agitation comprend un mélangeur statique et le procédé comprend les étapes de :
i) la fourniture :
   - d'un courant **A1** comprenant HF,
   - d'un courant **A2** comprenant 1,1,3,3-tetrachloropropene et/ou 1,3,3,3-tetrachloropropene, et
   - d'un réacteur contenant une phase liquide et comprenant un mélangeur statique,
i') la mise en contact dudit courant **A1** avec ledit courant **A2** dans ledit mélangeur statique pour former un mélange **B** comprenant HF, 1,1,3,3-tetrachloropropene et/ou 1,3,3,3-tetrachloropropene,
ii') la diffusion dudit mélange **B** dans ladite phase liquide pour mettre en œuvre l'étape ii) à partir de celui-ci.

Selon un mode de réalisation, ledit courant **A1** et ledit courant **A2** sont préchauffés avant la mise en œuvre de l'étape i') ou ii).

Selon un mode de réalisation préféré, l'étape ii) est mise en œuvre à une température comprise entre 20°C et 150°C.

Selon un mode de réalisation préféré, l'étape ii) est mise en œuvre à une pression comprise entre 1 et 20 bara.

Selon un mode de réalisation préféré, le procédé est mis en œuvre en continu dans un seul réacteur.

L'etape ii) est mise en œuvre en l'absence d'un catalyseur et la température de ladite phase liquide varie d'au maximum 2 °C.

Selon un mode de réalisation préféré, le courant **C** est purifié, de préférence par distillation, pour former un courant **C1** comprenant HCI et un courant **C2** comprenant 1-chloro-3,3,3-trifluoropropène et HF, ledit courant **C2** étant lui-même séparé en un courant **C3** comprenant 1-chloro-3,3,3-trifluoropropène et un courant **C4** comprenant majoritairement le HF recyclé à l'étape i') ou ii).

Selon un mode de réalisation préféré, ladite phase liquide est pauvre en HF, avantageusement ladite phase liquide comprend moins de 15% en poids de HF, de préférence moins de 10% en poids de HF, plus préférentiellement moins de 8% en poids de HF.

Selon un mode de réalisation préféré, le courant **C** est un courant gazeux.

### Brève description des figures

La figure 1 représente schématiquement un réacteur comprenant comme moyen d'agitation un mélangeur statique selon un mode de réalisation de la présente invention.
La figure 2 représente schématiquement un réacteur comprenant comme moyen d'agitation un dispositif de recirculation de la phase liquide selon un mode de réalisation de la présente invention.
La figure 3 représente schématiquement un réacteur comprenant comme moyen d'agitation un dispositif de reflux de HF dans la phase liquide selon un mode de réalisation de la présente invention.
La figure 4 représente schématiquement un réacteur comprenant comme moyen d'agitation un dispositif d'injection d'un gaz inerte selon un mode de réalisation de la présente invention.
La figure 5 représente schématiquement un réacteur comprenant comme moyen d'agitation un mélangeur statique et un dispositif de reflux de HF dans la phase liquide selon un mode de réalisation de la présente invention.
La figure 6 représente schématiquement un réacteur comprenant comme moyen d'agitation un mélangeur statique et un dispositif de recirculation de la phase liquide selon un mode de réalisation de la présente invention.
La figure 7 représente schématiquement un réacteur comprenant comme moyen d'agitation un mélangeur statique, un dispositif de reflux de HF dans la phase liquide et un dispositif de recirculation de la phase liquide selon un mode de réalisation de la présente invention.

### Description détaillée de l'invention

Selon un premier aspect de la présente invention un procédé de production de 1-chloro-3,3,3-trifluoropropène (HCFO-1233zd) est fourni. Le présent procédé comprend les étapes de :
i) la fourniture :
   - d'un courant **A1** comprenant HF,
   - d'un courant **A2** comprenant 1,1,3,3-tetrachloropropene et/ou 1,3,3,3-tetrachloropropene, et
   - d'un réacteur contenant une phase liquide,
ii) dans ledit réacteur, la mise en contact dans ladite phase liquide dudit courant **A1** comprenant HF avec ledit courant **A2** comprenant 1,1,3,3-tetrachloropropene et/ou 1,3,3,3-tetrachloropropene dans des conditions suffisantes pour produire un courant **C** comprenant 1-chloro-3,3,3-trifluoropropène, HF et HCl,
iii) la récupération dudit courant **C.**

L'étape ii) est mise en œuvre dans un réacteur pourvu de moyens d'agitation de ladite phase liquide. Lesdits moyens d'agitation sont aptes à maintenir la température de ladite phase liquide substantiellement constante au cours de l'étape ii). Le présent procédé permet, grâce au contrôle de la température de la phase liquide, la production de 1-chloro-3,3,3-trifluoropropène tout en minimisant la formation de coproduits surfluorés ou de composés lourds (dimères ou trimères de composés en C3).

Le terme « substantiellement constante » se réfère à une température variant d'au maximum 2°C en valeur absolue, plus préférentiellement d'au maximum 1,5°C en valeur absolue, en particulier d'au plus 1°C en valeur absolue, plus particulièrement d'au plus 0,5°C en valeur absolue.

Ainsi, pendant la mise en œuvre de l'étape ii), la température de ladite phase liquide varie d'au maximum 2°C en valeur absolue, plus préférentiellement d'au maximum 1,5°C en valeur absolue, en particulier d'au plus 1°C en valeur absolue, plus particulièrement d'au plus 0,5°C en valeur absolue.

La température de ladite phase liquide est mesurée en plusieurs points de celle-ci suivant les méthodes connues de l'homme du métier.

Selon un mode de réalisation particulier, ladite phase liquide pauvre en HF est une phase liquide comprenant moins de 15% en poids de HF, avantageusement moins de 10% en poids de HF, de préférence moins de 8% en poids de HF, plus préférentiellement moins de 6% en poids de HF, en particulier moins de 5% en poids de HF, plus particulièrement moins de 4% en poids de HF, de manière privilégiée moins de 2% en poids de HF sur base du poids total de ladite phase liquide.

Selon un mode de réalisation alternatif, ladite phase liquide est riche en HF. Avantageusement, dans ce mode de réalisation alternatif, ladite phase liquide comprend au moins 25% en poids de HF, de préférence au moins 30% en poids de HF, plus préférentiellement au moins 35% en poids de HF. Ainsi, ladite phase liquide peut comprendre au moins 25%, au moins 26%, au moins 27%, au moins 28%, au moins 29%, au moins 30%, au moins 31%, au moins 32%, au moins 33%, au moins 34%, au moins 35%, au moins 36%, au moins 37%, au moins 38%, au moins 39%, au moins 40%, au moins 41%, au moins 42%, au moins 43%, au moins 44%, au moins 45%, au moins 46%, au moins 47%, au moins 48%, au moins 49% ou au moins 50 % en poids de HF.

Le mode de réalisation dans lequel ladite phase liquide est pauvre en HF est néanmoins privilégié.

Ladite phase liquide peut comprendre au moins 10% en poids de composés de formule (I) C₃HₙFₘClₚ (I) dans laquelle n est un entier de 0 à 8, m est un entier de 0 à 8, et p est un entier de 0 à 8 ; de préférence n est un entier de 0 à 8, m est un entier de 0 à 6 et p est un entier de 0 à 6. Par exemple, des composés de formule (I) peuvent être C₃Cl₆, C₃H₄Cl₄ ou C₃H₃Cl₅. De préférence, ladite phase liquide peut comprendre au moins 10% en poids de composés de formule (I) C₃HₙFₘClₚ (I) dans laquelle n est un entier de 1 à 8, m est un entier de 0 à 4, et p est un entier de 0 à 4 ; de préférence n est un entier de 1 à 4, m est un entier de 0 à 3 et p est un entier de 2 à 4. Les composés de formule (I) peuvent être des composés de type propane ou propène comprenant un ou plusieurs atomes de chlore et/ou un ou plusieurs atomes de fluor. De préférence, ladite phase liquide peut comprendre au moins 10% en poids de composés de formule (I) sélectionnée parmi le groupe consistant en C₃H₂Cl₄, C₃H₂Cl₃F, C₃H₂Cl₂F₂, C₃H₃Cl₅, C₃H₃Cl₄F, C₃H₃Cl₃F₂ et C₃H₃Cl₂F₃. En particulier, ladite phase liquide peut comprendre au moins 10% en poids de composés de formule (I) sélectionnée parmi le groupe consistant en C₃H₂Cl₄, C₃H₂Cl₃F et C₃H₂Cl₂F₂. Ladite phase liquide peut comprendre au moins 15% en poids de composés de formule (I) C₃HₙFₘClₚ (I) dans laquelle n est un entier de 0 à 8, m est un entier de 0 à 8, et p est un entier de 0 à 8 ; de préférence n est un entier de 0 à 8, m est un entier de 0 à 6 et p est un entier de 0 à 6. En particulier, ladite phase liquide peut comprendre au moins 15% en poids de composés de formule (I) C₃HₙFₘClₚ (I) dans laquelle n est un entier de 1 à 8, m est un entier de 0 à 4, et p est un entier de 0 à 4; de préférence n est un entier de 1 à 4, m est un entier de 0 à 3 et p est un entier de 2 à 4. De préférence, ladite phase liquide peut comprendre au moins 15% en poids de composés de formule (I) sélectionnée parmi le groupe consistant en C₃H₂Cl₄, C₃H₂Cl₃F, C₃H₂Cl₂F₂, C₃H₃Cl₃, C₃H₃Cl₄F, C₃H₃Cl₃F₂ et C₃H₃Cl₂F₃. En particulier, ladite phase liquide peut comprendre au moins 15% en poids de composés de formule (I) sélectionnée parmi le groupe consistant en C₃H₂Cl₄, C₃H₂Cl₃F et C₃H₂Cl₂F₂. Ladite phase liquide peut comprendre au moins 20%, au moins 30%, au moins 40%, au moins 50%, au moins 60%, au moins 70%, au moins 80% ou au moins 90% en poids de composés de formule (I) C₃HₙFₘClₚ (I) dans laquelle n est un entier de 0 à 8, m est un entier de 0 à 8, et p est un entier de 0 à 8 ; de préférence n est un entier de 0 à 8, m est un entier de 0 à 6 et p est un entier de 0 à 6. Ladite phase liquide peut comprendre au moins 20%, au moins 30%, au moins 40%, au moins 50%, au moins 60%, au moins 70%, au moins 80% ou au moins 90% en poids de composés de formule (I) C₃HₙFₘClₚ (I) dans laquelle n est un entier de 1 à 8, m est un entier de 0 à 4, et p est un entier de 0 à 4; de préférence n est un entier de 1 à 4, m est un entier de 0 à 3 et p est un entier de 2 à 4. De préférence, ladite phase liquide peut comprendre au moins 20%, au moins 30%, au moins 40%, au moins 50%, au moins 60%, au moins 70%, au moins 80% ou au moins 90% en poids de composés de formule (I) sélectionnée parmi le groupe consistant en C₃H₂Cl₄, C₃H₂Cl₃F, C₃H₂Cl₂F₂, C₃H₃Cl₃, C₃H₃Cl₄F, C₃H₃Cl₃F₂ et C₃H₃Cl₂F₃. En particulier, ladite phase liquide peut comprendre au moins 20%, au moins 30%, au moins 40%, au moins 50%, au moins 60%, au moins 70%, au moins 80% ou au moins 90% en poids de composés de formule (I) sélectionnée parmi le groupe consistant en C₃H₂Cl₄, C₃H₂Cl₃F et C₃H₂Cl₂F₂.

De préférence, ledit courant **A2** comprend au moins 10% en poids de 1,1,3,3-tétrachloropropène et/ou 1,3,3,3-tetrachloropropene sur base du poids total dudit courant **A2.** Avantageusement, ledit courant **A2** comprend au moins 15% en poids de 1,1,3,3-tétrachloropropène et/ou 1,3,3,3-tetrachloropropene, de préférence au moins 20% en poids de 1,1,3,3-tétrachloropropène et/ou 1,3,3,3-tetrachloropropene, plus préférentiellement au moins 25% en poids de 1,1,3,3-tétrachloropropène et/ou 1,3,3,3-tetrachloropropene, en particulier au moins 30% en poids de 1,1,3,3-tétrachloropropène et/ou 1,3,3,3-tetrachloropropene, plus particulièrement au moins 35% en poids de 1,1,3,3-tétrachloropropène et/ou 1,3,3,3-tetrachloropropene, de manière privilégiée au moins 40% en poids de 1,1,3,3-tétrachloropropène et/ou 1,3,3,3-tetrachloropropene, de manière avantageusement privilégiée au moins 45% en poids de 1,1,3,3-tétrachloropropène et/ou 1,3,3,3-tetrachloropropene, de manière préférentiellement privilégiée au moins 50% en poids de 1,1,3,3-tétrachloropropène et/ou 1,3,3,3-tetrachloropropene, de manière particulièrement privilégiée au moins 55% en poids de 1,1,3,3-tétrachloropropène et/ou 1,3,3,3-tetrachloropropene sur base du poids total dudit courant **A2.**

De préférence, ledit courant **A2** comprend au moins 60% en poids ou au moins 65% en poids ou au moins 70% en poids ou au moins 75 % en poids ou au moins 80% en poids ou au moins 85% en poids ou au moins 90% en poids ou au moins 95% en poids ou au moins 99% en poids de 1,1,3,3-tétrachloropropène et/ou 1,3,3,3-tetrachloropropene sur base du poids total dudit courant **A2.**

Selon un mode de réalisation, ledit courant **A1** et ledit courant **A2** sont préchauffés avant la mise en œuvre de l'étape i') ou ii).

Selon un mode de réalisation préféré, l'étape ii) est mise en œuvre à une température comprise entre 20°C et 150°C, avantageusement entre 50°C et 150°C, de préférence entre 80 et 120°C, en particulier entre 90 et 110°C.

Selon un mode de réalisation préféré, l'étape ii) est mise en œuvre à une pression comprise entre 1 et 20 bara, avantageusement entre 5 et 20 bara, de préférence entre 5 et 18 bara, plus préférentiellement entre 7 et 18 bara, en particulier entre 10 et 18 bara, plus particulièrement entre 12 et 18 bara, de manière privilégiée entre 12 et 15 bara.

Selon un mode de réalisation préféré, le procédé est mis en œuvre en continu dans un seul réacteur.

Selon un mode de réalisation préféré, lesdits moyens d'agitation comprennent un mélangeur statique, un dispositif d'injection d'un gaz inerte dans ladite phase liquide, un dispositif de recirculation de la phase liquide, un dispositif d'agitation mécanique ou un dispositif de reflux de HF dans ladite phase liquide ou une combinaison de plusieurs de ces moyens d'agitation. De préférence, lesdits moyens d'agitation comprennent un mélangeur statique, un dispositif d'injection d'un gaz inerte dans ladite phase liquide, un dispositif de recirculation de la phase liquide ou un dispositif de reflux de HF dans ladite phase liquide ou une combinaison de plusieurs de ces moyens.

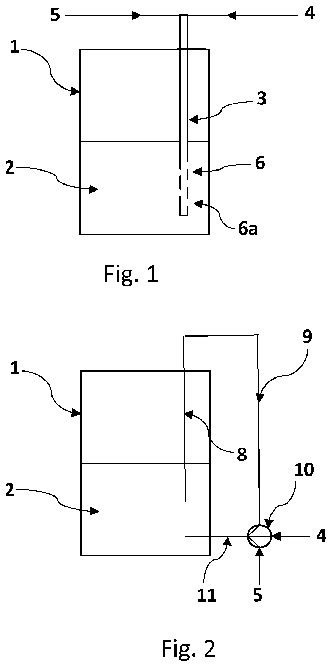

La figure 1 représente schématiquement un réacteur 1 comprenant comme moyen d'agitation un mélangeur statique. Dans la figure 1, le réacteur 1 comprend une phase liquide 2 pauvre en HF telle que définie dans la présente demande. Le HF et le HCFO-1230za sont introduits dans le réacteur respectivement par l'intermédiaire d'une ligne d'amenée 4 ou 5. Le HF et le HCFO-1230za sont mélangés dans le mélangeur statique 3 alimentant un dispositif de diffusion 6 équipé d'ouvertures 6a avant d'être introduit dans la phase liquide 2. Après avoir traversé le mélangeur statique 3, le dispositif de diffusion 6 permet la diffusion optimale du mélange de HF et de HCFO-1230za dans la phase liquide 2. Cette diffusion optimale permet une meilleure régulation de la température de la phase liquide 2. Ainsi, selon un mode de réalisation préféré, le réacteur dans lequel le présent procédé est mis en œuvre, peut comprendre un mélangeur statique et au moins une, de préférence au moins deux, ligne(s) d'amenée des courants **A1** et **A2.** Le mélangeur statique 3 apporte un mélange homogène vers le dispositif de diffusion 6 permettant la diffusion desdits courants **A1** et **A2** dans ladite phase liquide 2. Ainsi, le réacteur peut également comprendre un dispositif de diffusion équipé d'ouvertures et connecté audit mélangeur statique.

Selon un autre mode de réalisation, le réacteur 1 comprend comme moyen d'agitation un dispositif de recirculation de la phase liquide. Ce mode de réalisation est illustré à la figure 2. A la figure 2, le réacteur 1 comprend une phase liquide 2 pauvre en HF. Le HF et le HCFO-1230za sont introduits dans le réacteur respectivement par l'intermédiaire d'une ligne d'amenée 4 ou 5. L'introduction des réactifs, i.e. HF et HCFO-1230za, dans le réacteur 1 est effectuée via une pompe 10. La phase liquide 2 est extraite du réacteur 1 par la conduite 8 et acheminée vers la pompe 10 par la conduite 9. La pompe 10 permet l'introduction de la phase liquide soutirée par les conduites 8 et 9 dans la phase liquide 2 du réacteur 1. Cette introduction s'effectue par l'intermédiaire d'une conduite 11 connectant la pompe 10 au réacteur 1. Ainsi, selon un mode de réalisation préféré, le réacteur dans lequel le présent procédé est mis en œuvre, peut comprendre au moins une, de préférence au moins deux, ligne(s) d'amenée des courants **A1** et **A2.** Le réacteur peut également comprendre une pompe apte à soutirer une partie de ladite phase liquide contenue dans le réacteur et à permettre la recirculation de celle-ci dans le réacteur. Le réacteur peut également comprendre des conduites connectant ladite pompe avec la phase liquide du réacteur. Ainsi, le réacteur peut comprendre au moins une première conduite soutirant la phase liquide du réacteur et l'acheminant vers la pompe et au moins une seconde conduite connectant la pompe vers le réacteur et apte à introduire la phase liquide soutirée dans le réacteur. Lesdites lignes d'amenée des courants **A1** et **A2** peuvent être connectées à ladite pompe. Le réacteur peut également comprendre au moins une conduite soutirant un courant gazeux dudit réacteur pour l'acheminer vers ladite pompe.

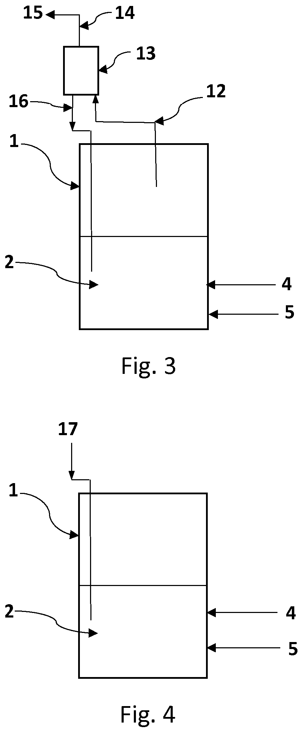

Selon un autre mode de réalisation, le réacteur 1 comprend comme moyen d'agitation un dispositif de reflux de HF dans la phase liquide. Ce mode de réalisation est illustré à la figure 3. A la figure 3, le réacteur 1 comprend une phase liquide 2 pauvre en HF. Le HF et le HCFO-1230za sont introduits dans la phase liquide 2 du réacteur 1 respectivement par l'intermédiaire d'une ligne d'amenée 4 ou 5. La phase gazeuse présente dans le ciel du réacteur 1 (par exemple le courant **C** selon la présente invention) est soutirée de celui-ci par la conduite 12 pour être acheminée vers un dispositif de distillation 13. Un courant 15 est récupéré en tête du dispositif de distillation 13 par l'intermédiaire de la conduite 14. Un courant 16 est récupéré en pied du dispositif de distillation 13 et est acheminé vers le réacteur 1 pour être introduit dans la phase liquide 2. Ainsi, selon un mode de réalisation préféré, le réacteur dans lequel le présent procédé est mis en œuvre, peut comprendre au moins une, de préférence au moins deux, ligne(s) d'amenée des courants **A1** et **A2.** Le réacteur peut également comprendre un dispositif de distillation, au moins une conduite alimentant ledit dispositif de distillation avec une phase gazeuse provenant du ciel dudit réacteur. Le dispositif de distillation comprend également une conduite connectant le pied du dispositif de distillation à la phase liquide du réacteur ; ladite conduite permettant de réintroduire un courant comprenant du HF dans ladite phase liquide du réacteur. Ledit dispositif de distillation peut comprendre également une conduite connecté en tête de celui-ci pour soutirer un courant comprenant 1-chloro-3,3,3-trifluoropropène.

Selon un autre mode de réalisation, le réacteur 1 comprend comme moyen d'agitation un dispositif d'injection d'un gaz inerte dans la phase liquide. Ce mode de réalisation est illustré à la figure 4. A la figure 4, le réacteur 1 comprend une phase liquide 2 pauvre en HF. Le HF et le HCFO-1230za sont introduits dans le réacteur respectivement par l'intermédiaire d'une ligne d'amenée 4 ou 5. Le gaz inerte est introduit dans la phase liquide via une conduite 17. Le gaz inerte peut être de l'azote, de l'argon ou de l'HCl. Ainsi, selon un mode de réalisation préféré, le réacteur dans lequel le présent procédé est mis en œuvre, peut comprendre au moins une, de préférence au moins deux, ligne(s) d'amenée des courants **A1** et **A2.** Le réacteur peut également comprendre une conduite d'amenée d'un gaz inerte plongeant dans ladite phase liquide contenu dans le réacteur.

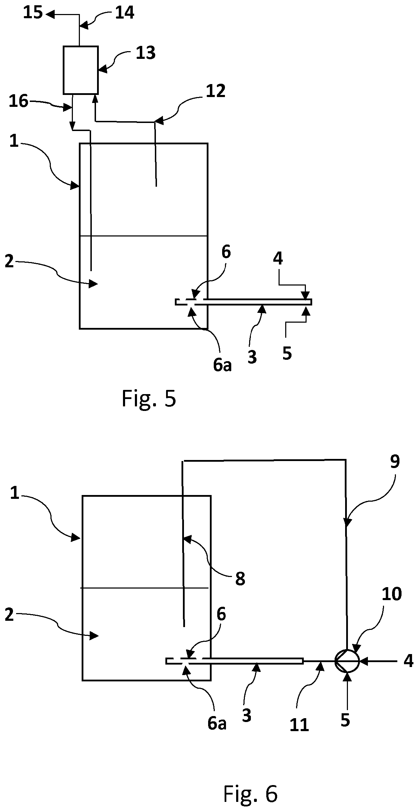

Selon un autre mode de réalisation, le réacteur 1 comprend comme moyen d'agitation un mélangeur statique et un dispositif de reflux du HF dans la phase liquide. Ce mode de réalisation est illustré à la figure 5. A la figure 5, le réacteur 1 comprend une phase liquide 2 pauvre en HF. Le HF et le HCFO-1230za sont introduits dans la phase liquide 2 du réacteur 1 respectivement à partir d'une ligne d'amenée 4 ou 5 alimentant un mélangeur statique 3, lui-même connecté à un dispositif de diffusion 6 équipé d'ouvertures 6a. La phase gazeuse présente dans le ciel du réacteur 1 (par exemple le courant **C** selon la présente invention) est soutirée de celui-ci par la conduite 12 pour être acheminée vers un dispositif de distillation 13. Un courant 15 est récupéré en tête du dispositif de distillation 13 par l'intermédiaire de la conduite 14. Un courant 16 est récupéré en pied du dispositif de distillation 13 et est acheminé vers le réacteur 1 pour être introduit dans la phase liquide 2. Ainsi, selon un mode de réalisation préféré, le réacteur dans lequel le présent procédé est mis en œuvre, peut comprendre au moins une, de préférence au moins deux, ligne(s) d'amenée des courants **A1** et **A2,** un mélangeur statique et optionnellement un dispositif de diffusion équipé d'ouvertures. Le mélangeur statique permet d'homogénéiser les courants **A1** et **A2** et de les diffuser dans ladite phase liquide à l'aide, de préférence du dispositif de diffusion équipé d'ouvertures. Le réacteur peut également comprendre un dispositif de distillation, au moins une conduite alimentant ledit dispositif de distillation avec une phase gazeuse provenant du ciel dudit réacteur. Le dispositif de distillation comprend également une conduite connectant le pied du dispositif de distillation à la phase liquide du réacteur ; ladite conduite permettant de réintroduire un courant comprenant du HF dans ladite phase liquide du réacteur. Ledit dispositif de distillation peut comprendre également une conduite connecté en tête de celui-ci pour soutirer un courant comprenant 1-chloro-3,3,3-trifluoropropène.

Selon un autre mode de réalisation, le réacteur 1 comprend comme moyen d'agitation un dispositif de recirculation de la phase liquide et un mélangeur statique. Ce mode de réalisation est illustré à la figure 6. A la figure 6, le réacteur 1 comprend une phase liquide 2 pauvre en HF. Le HF et le HCFO-1230za sont introduits dans le réacteur respectivement à partir des lignes d'amenée 4 ou 5. L'introduction des réactifs, i.e. HF et HCFO-1230za, dans le réacteur 1 est effectuée via une pompe 10. La phase liquide 2 est extraite du réacteur 1 par la conduite 8 et acheminée vers la pompe 10 par la conduite 9. La pompe 10 permet l'introduction de la phase liquide soutirée par les conduites 8 et 9 dans la phase liquide 2 du réacteur 1. La conduite 11 connecte la pompe 10 au réacteur 1 par l'intermédiaire du mélangeur statique 3 et du dispositif de diffusion 6 équipé d'ouvertures 6a. Une phase gazeuse peut également être présente dans le ciel du réacteur 1 (par exemple le courant **C** selon la présente invention). Ainsi, selon un mode de réalisation préféré, le réacteur dans lequel le présent procédé est mis en œuvre, peut comprendre au moins une, de préférence au moins deux, ligne(s) d'amenée des courants **A1** et **A2.** Le réacteur peut également comprendre un mélangeur statique. Le mélangeur statique permet d'homogénéiser les courants A1 et A2 et de les diffuser dans ladite phase liquide à l'aide, de préférence du dispositif de diffusion équipé d'ouvertures. Le réacteur peut également comprendre une pompe apte à soutirer une partie de ladite phase liquide contenue dans le réacteur et à permettre la recirculation de celle-ci dans le réacteur. Le réacteur peut également comprendre des conduites connectant ladite pompe avec la phase liquide du réacteur. Ainsi, le réacteur peut comprendre au moins une première conduite soutirant la phase liquide du réacteur et l'acheminant vers la pompe et au moins une seconde conduite connectant la pompe audit mélangeur statique. Lesdites lignes d'amenée des courants **A1** et **A2** peuvent être connectées à ladite pompe.

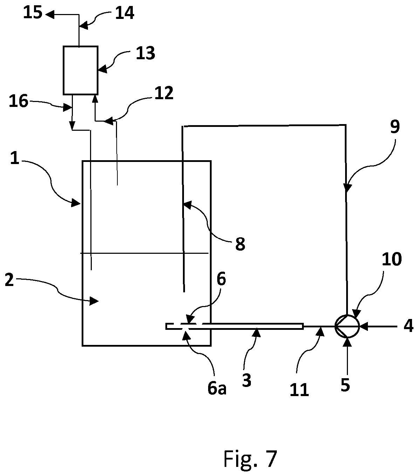

Selon un autre mode de réalisation, le réacteur 1 comprend comme moyen d'agitation un dispositif de recirculation de la phase liquide, un mélangeur statique et un dispositif de reflux du HF dans la phase liquide. Ce mode de réalisation est illustré à la figure 7. A la figure 7, le réacteur 1 comprend une phase liquide 2 pauvre en HF. Le HF et le HCFO-1230za sont introduits dans le réacteur respectivement à partir des lignes d'amenée 4 ou 5. L'introduction des réactifs, i.e. HF et HCFO-1230za, dans le réacteur 1 est effectuée via une pompe 10. La phase liquide 2 est extraite du réacteur 1 par la conduite 8 et acheminée vers la pompe 10 par la conduite 9. La pompe 10 permet l'introduction de la phase liquide soutirée par les conduites 8 et 9 dans la phase liquide 2 du réacteur 1. La conduite 11 connecte la pompe 10 au réacteur 1 par l'intermédiaire du mélangeur statique 3 et du dispositif de diffusion 6 équipé d'ouvertures 6a. La phase gazeuse présente dans le ciel du réacteur 1 est en partie soutirée de celui-ci par la conduite 12 pour être acheminée vers un dispositif de distillation 13. Un courant 15 est récupéré en tête du dispositif de distillation 13 par l'intermédiaire de la conduite 14. Un courant 16 est récupéré en pied du dispositif de distillation 13 et est acheminé vers le réacteur 1 pour être introduit dans la phase liquide 2. Ainsi, selon un mode de réalisation préféré, le réacteur dans lequel le présent procédé est mis en œuvre, peut comprendre au moins une, de préférence au moins deux, ligne(s) d'amenée des courants **A1** et **A2.** Le réacteur peut également comprendre un mélangeur statique. Le mélangeur statique permet d'homogénéiser les courants **A1** et **A2** et de les diffuser dans ladite phase liquide à l'aide, de préférence du dispositif de diffusion équipé d'ouvertures. Le réacteur peut également comprendre une pompe apte à soutirer une partie de ladite phase liquide contenue dans le réacteur et à permettre la recirculation de celle-ci dans le réacteur. Le réacteur peut également comprendre des conduites connectant ladite pompe avec la phase liquide du réacteur. Ainsi, le réacteur peut comprendre au moins une première conduite soutirant la phase liquide du réacteur et l'acheminant vers la pompe et au moins une seconde conduite connectant la pompe audit mélangeur statique. Lesdites lignes d'amenée des courants **A1** et **A2** peuvent être connectées à ladite pompe. Le réacteur peut également comprendre un dispositif de distillation, au moins une conduite alimentant ledit dispositif de distillation avec une phase gazeuse provenant du ciel dudit réacteur. Le dispositif de distillation comprend également une conduite connectant le pied du dispositif de distillation à la phase liquide du réacteur ; ladite conduite permettant de réintroduire un courant comprenant du HF dans ladite phase liquide du réacteur. Ledit dispositif de distillation peut comprendre également une conduite connecté en tête de celui-ci pour soutirer un courant comprenant 1-chloro-3,3,3-trifluoropropène.

Plus particulièrement, le moyen d'agitation comprend un mélangeur statique. Ainsi, le présent procédé de production de 1-chloro-3,3,3-trifluoropropène (HCFO-1233zd) comprend les étapes de :
i) la fourniture :
   - d'un courant **A1** comprenant HF,
   - d'un courant **A2** comprenant 1,1,3,3-tetrachloropropene et/ou 1,3,3,3-tetrachloropropene, et
   - d'un réacteur contenant une phase liquide et comprenant un mélangeur statique,
i') la mise en contact dudit courant **A1** avec ledit courant **A2** dans ledit mélangeur statique pour former un mélange **B** comprenant HF, 1,1,3,3-tetrachloropropene et/ou 1,3,3,3-tetrachloropropene,
ii') la diffusion dudit mélange **B** dans ladite phase liquide pour mettre en œuvre l'étape ii) à partir de celui-ci.

Ainsi, selon un mode de réalisation particulier, le présent procédé de production de 1-chloro-3,3,3-trifluoropropène (HCFO-1233zd) comprend les étapes de :
i) la fourniture :
   - d'un courant **A1** comprenant HF,
   - d'un courant **A2** comprenant 1,1,3,3-tetrachloropropene et/ou 1,3,3,3-tetrachloropropene, et
   - d'un réacteur contenant une phase liquide et comprenant un mélangeur statique,
i') mise en contact dudit courant **A1** avec ledit courant **A2** dans ledit mélangeur statique pour former un mélange **B** comprenant HF, 1,1,3,3-tetrachloropropene et/ou 1,3,3,3-tetrachloropropene,
ii') diffusion dudit mélange **B** dans ladite phase liquide,
ii) la mise en contact dans ladite phase liquide dudit courant **A1** comprenant HF avec ledit courant **A2** comprenant 1,1,3,3-tetrachloropropene et/ou 1,3,3,3-tetrachloropropene dans des conditions suffisantes pour produire un courant **C** comprenant 1-chloro-3,3,3-trifluoropropène, HF et HCl,
iii) la récupération dudit courant **C.**

L'utilisation d'un mélangeur statique permet un contrôle optimal de la température de la phase liquide pendant la mise en œuvre de l'étape ii). Ainsi dans ce mode de réalisation, la température de ladite phase liquide est substantiellement constante. De préférence, l'utilisation d'un mélangeur statique permet une variation de la température de la phase liquide d'au maximum 0,5°C.

De préférence, le mélangeur statique est combiné avec un ou plusieurs moyen(s) d'agitation tels que définis dans la présente demande. Ainsi, le réacteur peut comprendre, outre le mélangeur statique, un dispositif de reflux du HF dans la phase liquide et/ou un dispositif de recirculation de ladite phase liquide tel que défini dans la présente demande, notamment aux figures 5 à 7. Alternativement, le réacteur peut comprendre, outre le mélangeur statique, un dispositif d'injection d'un gaz inerte dans la phase liquide.

En outre, le mélangeur statique permet d'initier la réaction de fluoration entre l'acide fluorhydrique et le 1,1,3,3-tétrachloropropène et/ou le 1,3,3,3-tétrachloropropène. Dans ledit mélange **B,** le HF est sous forme gazeuse ou sous forme liquide. Dans ledit mélange **B,** le 1,1,3,3-tetrachloropropene et/ou 1,3,3,3-tetrachloropropene est sous forme liquide. Ledit mélange **B** peut également comprendre des composés ayant un degré de fluoration supérieur à celui du 1,1,3,3-tetrachloropropene ou du 1,3,3,3-tetrachloropropene. Le degré de fluoration correspond au nombre d'atomes de fluor contenu dans le composé considéré. Par exemple, le mélange **B** peut comprendre des composés trichlorofluoropropène (HCFO-1231) ou dichlorodifluoropropene (HCFO-1232). Il a été ainsi découvert de manière surprenante que l'utilisation de produits de départ tels que 1,1,3,3-tetrachloropropene et/ou 1,3,3,3-tetrachloropropene permettait une production du 1-chloro-3,3,3-trifluoropropène plus aisée et plus rapide dans des conditions opératoires moins contraignantes en terme de température et de pression. Ainsi, l'utilisation de produits de départ tels que 1,1,3,3-tetrachloropropene et/ou 1,3,3,3-tetrachloropropene en combinaison avec le mélangeur statique permet d'initier la réaction de fluoration dès leur mise en contact dans ledit mélangeur statique. L'HCl sous-produit lors de l'initiation de la réaction contribue à l'homogénéité du mélange des matières premières. Ainsi, la mise en œuvre du procédé est plus efficace qu'avec un produit de départ tel que le 1,1,1,3,3-pentachloropropane (HCC-240fa). Le mélangeur statique permet en outre de contrôler la diffusion du mélange **B** dans ladite phase liquide en limitant la formation de composés lourds (tels que mentionnés ci-dessus).

Le courant **C** est de préférence sous forme gazeuse. Le courant **C** peut également comprendre des composés organiques tels que les intermédiaires de la réaction de fluoration ou des coproduits. On peut citer notamment le dichlorodifluoropropene, le trichloromonofluoropropene, le fluorotetrachloropropane, le pentafluoropropane, le difluorotrichloropropane, le dichlorotrifluoropropane et le 1,3,3,3-tetrafluoropropene. De préférence, le courant **C** comprend 1,3,3,3-tetrafluoropropene et 1,1,1,3,3-pentafluoropropane. La teneur molaire en 1,3,3,3-tetrafluoropropene dans ledit courant **C** est inférieure à 0,2 mol%. La teneur molaire en 1,1,1,3,3-pentafluoropropane dans ledit courant **C** est inférieure à 0,5 mol%.

Selon un mode de réalisation préféré, le courant **C** est purifié, de préférence par distillation, pour former un courant **C1** comprenant HCI et un courant **C2** comprenant 1-chloro-3,3,3-trifluoropropène et HF, ledit courant **C2** étant lui-même séparé, par exemple par décantation à froid, en un courant **C3** comprenant 1-chloro-3,3,3-trifluoropropène et un courant **C4** comprenant HF recyclé à l'étape i') ou ii). L'étape de décantation à froid peut être mise en œuvre à une température de -50°C à 50°C, de préférence de -30°C à 0°C.

### EXEMPLES

Les exemples suivants illustrent l'invention sans la limiter. L'équipement utilisé consiste en un autoclave d'une capacité de 1 litre avec une double enveloppe, fabriqué en acier inoxydable 316L. Il est pourvu de moyens de mesure de température et de pression. Des ouvertures au sommet de l'autoclave permettent l'introduction des réactifs et l'enlèvement des produits. Un condenseur est prévu au sommet, ainsi qu'une vanne pour la régulation de la pression. Le condenseur est contrôlé en température au moyen d'un bain thermostaté. En raison de la régulation de pression, les produits de la réaction sont extraits au fur et à mesure. Ainsi, le flux de gaz de sortie passe dans un dispositif de lavage qui collecte les hydracides HF et HCl, puis est refroidi dans de l'azote liquide. La répartition molaire des produits du gaz de sortie est analysée périodiquement, par GPC (chromatographie en phase gazeuse). A la fin de l'essai, le milieu réactionnel est refroidi à température ambiante puis dépressurisé.

### Exemple 1 comparatif : fluoration en phase liquide du 1230za sans agitation

Une quantité de 150 g d'HF est introduite dans l'autoclave. La température du réacteur est ajustée à 90°C dans la phase liquide. La régulation de la pression est effectuée à 13 bara. Une fois la température stabilisée, 135 g de 1230za sont introduits dans le réacteur. Le rapport molaire d'HF sur le composé organique est donc de 10. La température de la phase liquide prise en même temps en deux points est différente. Après deux heures de réaction, l'essai est arrêté et traité selon les modalités décrites précédemment. La conversion de HCO-1230za est de 69,2%. La quantité de composés lourds formée est estimée par la masse de produits organiques (autre que 1230za) récupérée du réacteur en fin de réaction divisée par la masse de 1230za ayant réagi. Celle-ci est de 9% en poids.

### Exemple 2 : fluoration en phase liquide du 1230za avec agitation

Une quantité de 150 g d'HF est introduite dans l'autoclave. Le réacteur est équipé d'un moyen d'agitation mécanique. La température du réacteur est ajustée à 90°C dans la phase liquide. La régulation de la pression est effectuée à 13 bara. Une fois la température stabilisée, 136 g de 1230za sont introduits dans le réacteur. Le rapport molaire d'HF sur le composé organique est donc de 10. La température de la phase liquide prise en même temps en deux points différents ne varie pas. Après deux heures de réaction, l'essai est arrêté et traité selon les modalités décrites précédemment. La conversion de HCO-1230za est de 100%. La quantité de composés lourds formée est estimée par la masse de produits organiques (autre que 1230za) récupérée du réacteur en fin de réaction divisée par la masse de 1230za ayant réagi. Celle-ci est de 6% en poids.

La présence d'un moyen d'agitation tel que prévu par la présente invention permet de diminuer la quantité de composés lourds formés pendant la réaction de fluoration. Comme démontré par l'exemple 2, la présence d'un moyen d'agitation permet de diminuer la teneur en composés lourds à 6% en poids contre 9% à l'exemple 1 mis en œuvre en l'absence d'agitation.

### Exemple 3 : fluoration en phase liquide du 1230za avec un mélangeur statique

L'équipement pilote utilisé consiste en un réacteur d'une capacité de 60 litres en acier inoxydable 316L. Il est pourvu de moyens de mesure de température, de pression et de niveau liquide. Les deux réactifs sont préchauffés. Ils sont ensuite mélangés et alimentés dans le réacteur à l'aide d'un tube plongeur équipé d'un mélangeur statique puis d'un diffuseur à l'extrémité. Un système de prélèvement en ligne permet d'échantillonner le flux de gaz de sortie qui est ainsi orienté vers un appareil de chromatographie gazeuse. Les réactifs sont alimentés en continu et les produits sont analysés et collectés en continu. Une quantité de 25 litres de 1230za est introduite dans le réacteur. La régulation de pression est ajustée à 15 bara. Les réactifs sont ensuite alimentés avec les débits suivants : 2,3 kg/h de HF et 3,2 kg/h de 1230za. La température de la phase liquide prise en même temps en cinq points différents après 24 h de fonctionnement en continu du réacteur varie au maximum de 0,5°C. La composition du flux organique gazeux résultant est donnée : 95,4 mol% de HFCO-1233zdE, 4,17mol% de HCFO-1233zdZ, 0,14mol% de 1234ze (E+Z), 0,002 mol% de 245fa.

### Exemple 4 (comparatif) : fluoration en phase liquide du 1230za sans mélangeur statique

L'exemple 3 est reproduit sans mélangeur statique. Les deux réactifs alimentent indépendamment le réacteur à l'aide de deux tubes plongeurs. La température de la phase liquide prise en même temps en cinq points différents du réacteur varie entre 89°C à la surface liquide et 94°C au fond du réacteur. La composition du flux organique gazeux résultant est donnée : 94,6 mol% de HFCO-1233zdE, 4,53 mol% de HCFO-1233zdZ, 0,18 mol% de 1234ze (E+Z), 0,025 mol% de 245fa.

La présence d'un mélangeur statique comme moyen d'agitation permet également de diminuer la teneur en coproduits. En effet, la teneur en 1234ze et en 245fa diminue lorsque le procédé de fluoration est mis en œuvre en présence d'un moyen d'agitation tel que le mélangeur statique.

## Revendications

1. Procédé de production de 1-chloro-3,3,3-trifluoropropène (HCFO-1233zd) comprenant les étapes de :
i) la fourniture :
• d'un courant **A1** comprenant HF,
• d'un courant **A2** comprenant 1,1,3,3-tetrachloropropene et/ou 1,3,3,3-tetrachloropropene, et
• d'un réacteur contenant une phase liquide,
ii) dans ledit réacteur, la mise en contact dans ladite phase liquide et en l'absence d'un catalyseur dudit courant **A1** comprenant HF avec ledit courant **A2** comprenant 1,1,3,3-tetrachloropropene et/ou 1,3,3,3-tetrachloropropene dans des conditions suffisantes pour produire un courant **C** comprenant 1-chloro-3,3,3-trifluoropropène, HF et HCl,
iii) la récupération dudit courant **C,**
**caractérisé en ce que** ledit réacteur est pourvu de moyens d'agitation de ladite phase liquide de sorte à ce que pendant la mise en œuvre de l'étape ii), la température de ladite phase liquide varie d'au maximum 2°C.

2. Procédé selon la revendication précédente **caractérisé en ce que** lesdits moyens d'agitation comprennent un mélangeur statique, un dispositif d'injection dans ladite phase liquide d'un gaz inerte, un dispositif de recirculation de la phase liquide ou un dispositif de reflux de HF dans ladite phase liquide.

3. Procédé selon l'une quelconque des revendications précédentes **caractérisé en ce que** le moyen d'agitation comprend un mélangeur statique et le procédé comprend les étapes de :
i) la fourniture :
• d'un courant **A1** comprenant HF,
• d'un courant **A2** comprenant 1,1,3,3-tetrachloropropene et/ou 1,3,3,3-tetrachloropropene, et
• d'un réacteur contenant une phase liquide et comprenant un mélangeur statique,
i') la mise en contact dudit courant **A1** avec ledit courant **A2** dans ledit mélangeur statique pour former un mélange **B** comprenant HF, 1,1,3,3-tetrachloropropene et/ou 1,3,3,3-tetrachloropropene,
ii') la diffusion dudit mélange **B** dans ladite phase liquide pour mettre en œuvre l'étape ii) à partir de celui-ci.

4. Procédé selon l'une quelconque des revendications précédentes **caractérisé en ce que** ledit courant **A1** et ledit courant **A2** sont préchauffés avant la mise en œuvre de l'étape i') ou ii).

5. Procédé selon l'une quelconque des revendications précédentes **caractérisé en ce que** l'étape ii) est mise en œuvre à une température comprise entre 20°C et 150°C.

6. Procédé selon l'une quelconque des revendications précédentes **caractérisé en ce que** l'étape ii) est mise en œuvre à une pression comprise entre 1 et 20 bara.

7. Procédé selon l'une quelconque des revendications précédentes **caractérisé en ce qu'**il est mis en œuvre en continu dans un seul réacteur.

8. Procédé selon l'une quelconque des revendications précédentes **caractérisé en ce que** le courant **C** est purifié, de préférence par distillation, pour former un courant **C1** comprenant HCI et un courant **C2** comprenant 1-chloro-3,3,3-trifluoropropène et HF, ledit courant **C2** étant lui-même séparé en un courant **C3** comprenant 1-chloro-3,3,3-trifluoropropène et un courant **C4** comprenant majoritairement le HF recyclé à l'étape i') ou ii).

9. Procédé selon l'une quelconque des revendications précédentes **caractérisé en ce que** ladite phase liquide est pauvre en HF, avantageusement ladite phase liquide comprend moins de 15% en poids de HF, de préférence moins de 10% en poids de HF, plus préférentiellement moins de 8% en poids de HF.

10. Procédé selon l'une quelconque des revendications précédentes **caractérisé en ce que** le courant **C** est un courant gazeux.

## Patentansprüche

1. Verfahren zur Herstellung von 1-Chlor-3,3,3-trifluorpropen (HCFO-1233zd), das die folgenden Schritte umfasst:
i) die Bereitstellung:
• eines HF umfassenden Stroms A1,
• eines 1,1,3,3-Tetrachlorpropen und/oder 1,3,3,3-Tetrachlorpropen umfassenden Stroms A2 und
• eines Reaktors, der eine flüssige Phase enthält,
ii) in dem Reaktor das In-Kontakt-Bringen in der flüssigen Phase und in Abwesenheit eines Katalysators des HF umfassenden Stroms A1 mit dem 1,1,3,3-Tetrachlorpropen und/oder 1,3,3,3-Tetrachlorpropen umfassenden Strom A2 unter Bedingungen, die ausreichend sind, um einen 1-Chlor-3,3,3-trifluorpropen, HF und HCl umfassenden Strom C zu erzeugen,
iii) das Gewinnen des Stroms C,
**dadurch gekennzeichnet, dass** der Reaktor Einrichtungen zum Rühren der flüssigen Phase so aufweist, dass sich die Temperatur der flüssigen Phase während der Durchführung von Schritt ii) um maximal 2 °C ändert.

2. Verfahren nach dem vorhergehenden Anspruch, **dadurch gekennzeichnet, dass** die Rühreinrichtungen einen statischen Mischer, eine Vorrichtung zum Einspritzen eines Inertgases in die flüssige Phase, eine Vorrichtung zur Rückführung der flüssigen Phase oder eine Vorrichtung zum Halten von HF unter Rückfluss in der flüssigen Phase umfassen.

3. Verfahren nach einem der vorhergehenden Ansprüche, **dadurch gekennzeichnet, dass** die Rühreinrichtung einen statischen Mischer umfasst und das Verfahren die folgenden Schritte umfasst:
i) die Bereitstellung:
• eines HF umfassenden Stroms A1,
• eines 1,1,3,3-Tetrachlorpropen und/oder 1,3,3,3-Tetrachlorpropen umfassenden Stroms A2 und
• eines Reaktors, der eine flüssige Phase enthält und einen statischen Mischer umfasst,
i') das In-Kontakt-Bringen des Stroms A1 mit dem Strom A2 in dem statischen Mischer, wodurch eine HF, 1,1,3,3-Tetrachlorpropen und/oder 1,3,3,3-Tetrachlorpropen umfassende Mischung B gebildet wird,
ii') das Verteilen der Mischung B in der flüssigen Phase, wodurch davon ausgehend Schritt ii) durchgeführt wird.

4. Verfahren nach einem der vorhergehenden Ansprüche, **dadurch gekennzeichnet, dass** der Strom A1 und der Strom A2 vor der Durchführung von Schritt i') oder ii) vorgewärmt werden.

5. Verfahren nach einem der vorhergehenden Ansprüche, **dadurch gekennzeichnet, dass** Schritt ii) bei einer Temperatur zwischen 20 °C und 150 °C durchgeführt wird.

6. Verfahren nach einem der vorhergehenden Ansprüche, **dadurch gekennzeichnet, dass** Schritt ii) bei einem Druck zwischen 1 und 20 bara durchgeführt wird.

7. Verfahren nach einem der vorhergehenden Ansprüche, **dadurch gekennzeichnet, dass** es in einem einzelnen Reaktor kontinuierlich durchgeführt wird.

8. Verfahren nach einem der vorhergehenden Ansprüche, **dadurch gekennzeichnet, dass** Strom C vorzugsweise durch Destillation gereinigt wird, wodurch ein HCl umfassender Strom C1 und ein 1-Chlor-3,3,3-trifluorpropen und HF umfassender Strom C2 gebildet werden, wobei der Strom C2 selbst in einen 1-Chlor-3,3,3-trifluorpropen umfassenden Strom C3 und einen Strom C4 getrennt wird, der überwiegend HF umfasst, das in Schritt i') oder ii) zurückgeführt wird.

9. Verfahren nach einem der vorhergehenden Ansprüche, **dadurch gekennzeichnet, dass** die flüssige Phase arm an HF ist, wobei die flüssige Phase vorteilhafterweise weniger als 15 Gew.-% HF, vorzugsweise weniger als 10 Gew.-% HF, stärker bevorzugt weniger als 8 Gew.-% HF umfasst.

10. Verfahren nach einem der vorhergehenden Ansprüche, **dadurch gekennzeichnet, dass** es sich bei dem Strom C um einen Gasstrom handelt.

## Claims

1. Process for producing 1-chloro-3,3,3-trifluoropropene (HCFO-1233zd), comprising the steps of:
i) providing:
• a stream **A1** comprising HF,
• a stream **A2** comprising 1,1,3,3-tetrachloropropene and/or 1,3,3,3-tetrachloropropene, and
• a reactor containing a liquid phase,
ii) in said reactor, contacting, in said liquid phase and in the absence of a catalyst, said stream **A1** comprising HF with said stream **A2** comprising 1,1,3,3-tetrachloropropene and/or 1,3,3,3-tetrachloropropene under conditions which are sufficient to produce a stream **C** comprising 1-chloro-3,3,3-trifluoropropene, HF and HCl,
iii) recovering said stream **C,**
**characterized in that** said reactor is provided with means for stirring said liquid phase such that, during the implementation of step ii), the temperature of said liquid phase varies by a maximum of 2°C.

2. Process according to the preceding claim, **characterized in that** said stirring means comprise a static mixer, a device for injecting an inert gas into said liquid phase, a device for recirculating the liquid phase, or a device for returning a flow of HF into said liquid phase.

3. Process according to either one of the preceding claims, **characterized in that** the stirring means comprises a static mixer and the process comprises the steps of:
i) providing:
• a stream **A1** comprising HF,
• a stream **A2** comprising 1,1,3,3-tetrachloropropene and/or 1,3,3,3-tetrachloropropene, and
• a reactor containing a liquid phase and comprising a static mixer,
i') contacting said stream **A1** with said stream **A2** in said static mixer to form a mixture **B** comprising HF, 1,1,3,3-tetrachloropropene and/or 1,3,3,3-tetrachloropropene,
ii') diffusing said mixture **B** into said liquid phase in order to carry out step ii) proceeding from this.

4. Process according to any one of the preceding claims, **characterized in that** said stream **A1** and said stream **A2** are preheated before carrying out step i') or ii).

5. Process according to any one of the preceding claims, **characterized in that** step ii) is carried out at a temperature of between 20°C and 150°C.

6. Process according to any one of the preceding claims, **characterized in that** step ii) is carried out at a pressure of between 1 and 20 bara.

7. Process according to any one of the preceding claims, **characterized in that** it is carried out in continuous mode in a single reactor.

8. Process according to any one of the preceding claims, **characterized in that** stream **C** is purified, preferably by distillation, to form a stream **C1** comprising HCl and a stream **C2** comprising 1-chloro-3,3,3-trifluoropropene and HF, said stream **C2** itself being separated into a stream **C3** comprising 1-chloro-3,3,3-trifluoropropene and a stream **C4** predominantly comprising the HF recycled to step i') or ii).

9. Process according to any one of the preceding claims, **characterized in that** said liquid phase is low in HF, advantageously said liquid phase comprises less than 15% by weight of HF, preferably less than 10% by weight of HF, more preferentially less than 8% by weight of HF.

10. Process according to any one of the preceding claims, **characterized in that** stream **C** is a gaseous stream.
